# EUROPEAN PATENT APPLICATION

(11) **EP 0 661 267 A1**
(43) Date of publication of application: **05.07.1995**
(21) Application number: 95200465.3
(22) Date of filing: 23.05.1990
(51) Int. Cl.: C07D 211/56, A61K 47/48

(54) **Chelating agent for attaching metal ions to proteins**

(30) Priority: 26.05.1989 US 358917
(62) Divisional of application: 90914273.9
(71) Applicant: Akzo Nobel N.V., NL-6824 BM Arnhem (NL)
(72) Inventor: Ramaswamy, Subramanian, Frederick-Maryland 21701 (US)
(74) Representative: Hermans, F.G.M.

(57) **Abstract**

New bifunctional chelating agents useful for attaching metal ions to proteins, polypeptides and other polymers and methods for their preparation are described. These reagent are unique in their ability to bind a variety of metal ions and to yield a high metal ion concentration per protein molecule. Protein metal chelates thus obtained will have useful radiophysical, chemical, fluorescent, photochemical and magnetic properties suitable for biomedical applications.

## Description

This invention relates to new chelating agents for attaching metal ions to proteins such as albumin, transferrin, antibodies and etc.

### BACKGROUND OF THE INVENTION

Attachment of metal ions to proteins leads to several useful products. These include fluorescent, radioactive and paramagnetic metal ions attached proteins that can be used as probes in vivo in biological systems and in vitro in analytical systems such as radioimmunoassays. For example attachment of radionuclides to monoclonal antibodies that recognize tumor associated antigens provides radioimmunoconjugates useful for cancer diagnosis and therapy. The monoclonal antibodies are used as carriers of desired substances to specific sites in vivo. Several chelating agents such as diethylenetriaminepentaacetic acid (DTPA), ethylene-diaminetetraacetic acid (EDTA) and macrocyclics have been reported to form stable complexes when attached to protein. However, kinetic instability of the radioimmunoconjugate or the chelate under physiological conditions results in the breakdown of these complexes. Despite several attempts to modify the mode of binding, structure of chelate and etc., in vivo administration of such radio-immunoconjugates results in accumulation of radio-activity in non-target tissues, particularly the liver. Hence, there is an obvious need for new chelating agents for binding radiometals to antibodies forming complexes that do not disassociate when administered to a patient.

### SUMMARY OF THE INVENTION

It is an object of this invention to provide new chelating agents for attaching metal ions to proteins and thereby provide an aqueous solution containing antibody-chelate conjugate that is stable in vivo.

It is further an object of this invention to provide chelating agents to bind a variety of metal ions, including In, Y, Gd, Tb, Cu, Co, Sm, Rh, Ru, Re, Tc, Fe, Pb, Ba, actinides, lanthanides and transition metal ions.

It is further an object of this invention to synthesize new chelation structures useful for attaching metal ions to proteins, including monoclonal antibodies.

It is another object of this invention to obtain versatile chelating agents that are not only suitable for binding to low molecular weight proteins such as albumin and IgG, but also to high molecular weight proteins such as IgM (9x10⁵), lipoproteins (2x10⁶) and etc.

It is still another object of this invention to obtain an improved method for preparing metal chelate conjugated antibodies.

An additional object of this invention is to obtain a chelation structure that provides a high metal ion concentration per antibody molecule without destroying the biological activity of the conjugated protein to a significant extent.

It is still another object of this invention to obtain fluorescent labeled proteins by attaching fluorescent/luminescent metal ions to protein-chelate conjugates.

It is further the object of this invention to obtain metal ion binding reagents that can be attached to chromatographic column materials such as polymers and gels, forming chelate affinity columns.

These and other objects are accomplished by one or more of the embodiments of the present invention.

### BRIEF DESCRIPTION OF THE DRAWINGS

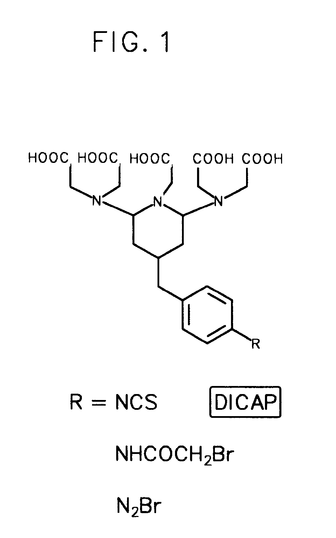

The bifunctional chelating agents of the invention are shown in Figure 1.

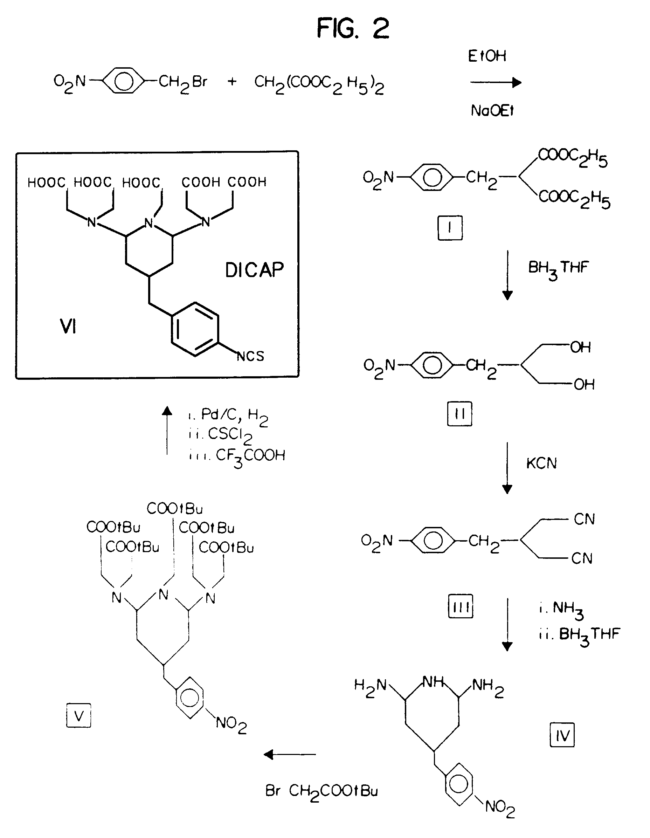

Figure 2 illustrates synthetic procedures for obtaining the metal-binding chelating agent DICAP.

### DESCRIPTION OF PREFERRED EMBODIMENTS

One embodiment of this invention is directed to a buffered solution containing an antibody or protein chelate conjugate wherein all the metal ions are bound to protein via the chelator.

Attachment of radiometals to antibodies requires first the attachment of ligands to antibodies. Ligands, also termed chelators or chelating agents, are compounds that can bind radiometals efficiently through the formation of coordinate bonds. Ligands such as polyaminocarboxylic acids, macrocyclics, crown ethers, cryptands and cyclams can be used to bind radiometals. The choice of the ligand depends on the choice of the radionuclide involved and the properties of the protein conjugated. For radioimmunoimaging and therapy the radionuclides of interest include ¹¹¹In, ⁹⁰Y, ²⁰³Pb, ¹⁵⁵Sm, ¹⁰¹Rh, ⁹⁷Ru, ⁶⁷Cu, ²⁰¹Tl, ⁹⁹mTc, ⁵⁵Co, ¹⁸⁶Re, ¹⁸⁸Re, ²¹²Bi and ²¹³Bi. For paramagnetic metal ions, Gd and other similar metal ions can be used. For fluorescence or luminescence based investigations, metal ions such as Tb, Eu, Ru and Rh can be employed.

Ligands suitable for attachment to both proteins and metal ions are often called bifunctional chelating agents. In addition to having a metal binding moiety, these compounds also possess reactive functional groups useful for attachment to proteins. The reactive functional groups are already known in the art. Examples of these groups are isothiocyanato, bromo-acetamido, diazo, N-hydroxysuccinimide esters and anhydrides. These groups can be incorporated into chelating agents. The metal ions can be part of a linker molecule containing several carbon atoms [(CH₂)n] and several other groups. Some of the factors to be considered while constructing an immunoconjugate are: (1) They must be stable, (2) readily bind the metal ions and (3) retain the biological activity of the protein. The chelation structure described in this invention satisfies these conditions.

In addition to the chelating agents, this invention comprises the synthesis of a family of bifunctional chelating agents starting from (p)-nitro-benzyl bromide and diethylmalonate. From the condensed products, several amines such as (p)NO₂Ph-CH₂-CH(CH₂NH₂)₂, (p)NO₂-Ph-CH-(CH₂.CH₂.NH₂)₂ and 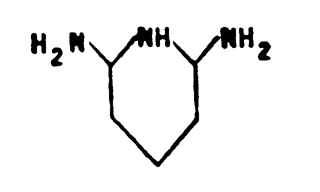
with (p)NO₂-Ph-CH₂ at C-3 can be prepared. The latter product is the key intermediate from which the metal binding chelating agents in Figure 1 can be synthesized. These reagents also possess a reactive functional group such as -NCS, -NH-CO-CH₂Br, or -N₂Br, which can be used to attach them to proteins.

Conventional chelating agents lead to several different products when coupled with proteins (e.g. aggregates). Often use of linkers such as dianhydrides may be the reason for this. By employing linkers such as isothiocyanate or -NH-CO-CH₂Br, these complications are avoided.

The chelatic agents can be coupled to proteins by gentle and simple means. One procedure involves incubating the protein and chelating agent in a suitable buffer at pH 6-9 for 1-3 hrs at 37 °C, and purifying the protein-chelate conjugate formed by gel filtration chromatography. We coupled these reagents to human serum albumin and the resultant chelate conjugated albumin binds metals such as ⁵⁷Co with very high efficiency (>95%). The proteins can be monoclonal or polyclonal antibodies, or other proteins such as transferrin, albumin and derivatives thereof. One can modify the linkers in order to obtain maximum immuno-reactivity. The metal ion can be transition metal ions, paramagnetic, radioactive and fluorescent/luminescent metal ions, lanthanides, actinides and others. These include but are not limited to Gd, Tb, Eu, Ru, Rh, Pd, Pb, Sm, Tb, Ga, In, Tl, Fe, Co, Ni, Re, Y, Tc, Cr, Os, Ir, Sb and Cu. The procedure for attaching the metal ions involves incubation of the immunoconjugate with a metal salt solution at pH 5-9, at temperatures of 20-37 °C for a period of 0.5-6 hrs., or at 4 °C for 24 hours, followed by gel filtration chromatography.

If the metal ion involved is not radioactive, the metal-chelate conjugate can be prepared and purified first, and then attached to the protein to obtain metalchelate-protein conjugate.

The reagents in Figure 1 are polyaminocarboxylates that will bind radiometals and other metal ions, and can be attached to proteins such as monoclonal antibodies.

The presence of unbound metal ions, if any, in the purified conjugate can be determined using standard analytical techniques such as thin layer chromatography (ascending), NMR, IR, or visible or fluorescence spectroscopy.

### Example 1

### Synthesis of DICAP:

The synthesis of DICAP is described in Figure 2. p-Nitrobenzylbromide was condensed with diethylmalonate to obtain p-nitrobenzyl diethylmalonate (I), which was reduced to a diol (II), converted to a dinitrile (III) and then converted to a triamine (IV). Carboxymethylation followed by reduction and treatment with CSCl₂ and CF₃COOH yielded DICAP (VI).

### A. 2-(4-nitrobenzyl)diethylmalonate (I):

6.9 g (0.3 mole) of sodium metal was added gradually to a stirred solution of 300 ml absolute ethanol under a nitrogen atmosphere. After all metal had dissolved 96 g (0.6 mole) diethylmalonate was slowly added dropwise to the solution. This was followed by the addition of 65 g (0.3 mole) of 4-nitrobenzylbromide in portions over one hour. The solution was heated to reflux for 24 hrs., and precipitated by-product was filtered off. The solution was evaporated and the crystallized product was filtered and washed with ethanol. Yield 34.9 g (40%), m.pt. 60 °C Ir 1736 cm⁻¹, 1524 cm⁻¹, 1346 cm⁻¹, 1151 cm⁻¹, 852 cm⁻¹, KBr pellet. NMR, CDCl₃, 8.14, d, 2H; 7.37, d, 2H; 4.15, q, 4H; 3.64, t, 1H; 3.30, d, 2H; 1.22, t, 6H.

### B. 2-(4-nitrobenzyl)-1,3-propanediol (II):

11.3 g (0.038 mole) (I) was dissolved in 20 ml anhydrous THF and injected into a stirred solution of 190 ml 1M BH₃-THF solution under a nitrogen atmosphere at 4 °C. The solution was slowly heated to reflux for 18 hours. The solution was then cooled to room temperature and methanol was added in small portions to quench the excess BH₃ complex. The solution was then rotoevaporated. Further purification was performed by silica gel chromatography. Yield was 6.3 g (78%), m.pt. 85 °C. IR (KBr pellet) 3260 cm⁻¹, 1539 cm⁻¹, 1351 cm⁻¹, 1030 cm⁻¹. NMR 3.73 ppm, d, 4H; 2.78 ppm, d, 2H; 2.35 ppm, s, 2H; 2.03 ppm, s, 1H.

### C. 3-(4-nitrobenzyl)1,5-pentanedinitrile (III):

6.9 g (0.036 mole) p-toluenesulfonylchloride (tosyl) was dissolved in 10 ml dry pyridine and cooled to 4 °C. 2.5 g (0.012 mole) II was diluted in 5 ml dry pyridine and added slowly to the stirred tosyl solution with cooling. The solution was stirred for 15 min. at 4 °C, then allowed to warm to room temperature and stirred for 3 hrs. The reaction was quenched by pouring the solution slowly with stirring into 100 ml of 30% HCl at 4 °C. The product was extracted with CH₂Cl₂, evaporated and crystallized in ethanol to produce the tosyl derivative. m.pt. 85-89 °C. IR (KBr pellet) 1524 cm⁻¹, 1352 cm⁻¹, 1194 cm⁻¹, 1176 cm⁻¹.

3.1 g KCN was suspended in a stirred solution of 30 ml absolute ethanol at 4 °C. The above tosyl derivative was dissolved in a 1:1 mixture of CH₂Cl₂:EtOH and added to the KCN mixture. The solution was slowly heated to reflux for 24 hours. The solution was then rotoevaporated, extracted with CH₂Cl₂, filtered and evaporated. The oil was then run down a silica gel column to be isolated as an orange oil (III). 1.9 g Yield 70%; IR (NaCl plate) 2248 cm⁻¹; NMR 2.96 ppm, s, 2H; 2.5 ppm, s, 4H; 2.13 ppm, m, 1H.

### D. (4-nitrobenzyl)glutarimidine (IV):

2.2 g (0.0096 mole) III was dissolved in 100 ml dry methanol. Ammonia gas was bubbled through the solution to saturation. The solution was added to a pressure reactor (bomb), sealed and heated at a pressure of 80-100 psi for 15 hrs. The bomb was cooled to ambient temperature, the reaction mixture removed and the solvent evaporated to a thick dark oil. IR (NaCl plates) 1666 cm⁻¹.

The above product was dissolved in 25 ml of anhydrous THF (tetrahydrofuran) and added to 75 ml 1M BH3.THF (borane tetrahydrofuran) solution at 4 °C under a nitrogen atmosphere. The solution was then allowed to warm to room temperature and stirred at 25 °C for 4 days. Thereafter, the reaction was quenched with methanol and the solution concentrated by evaporation. The oil was dissolved in CH₂Cl₂ and HCl gas was bubbled through the solution to precipitate the product. IR (KBr pellet) 1604 cm⁻¹, 3418 cm⁻¹. The product was fluorescamine positive showing the presence of a primary amine. It also yielded a positive test for a secondary amine.

### E. 2,6-diamino-4-(4-nitrobenzyl)-1-azacyclohexyl-N,N',N',N'',N''-pentaacetic acid tert-butyl ester (V):

54 mg (IV) was dissolved in 3 ml dry methanol. Triethylamine was added, followed by 250 µl of tertbutyl bromoacetate. The solution was evaporated to dryness and then run down a silica gel column with CH₂Cl₂ and a gradient increase in methanol content. Yield 25%. NMR showed ratios of benzyl H's to t-butyl H's 4:44.2 (calculated 4:45); tert-butyl H's 1.47 ppm, 3 distinct esters ratio 2:2:1; carboxymethyl H's 3.40 ppm, 9.6 H's (theoretical-10).

### F. DICAP (VI):

15 mg (V) was dissolved in 5 ml methanol and cooled to 4 °C. A catalytic amount of 10% palladium-carbon was added with stirring. H₂ gas was then bubbled through the stirred reaction mixture at 4 °C. After 10 min the solution was warmed to 25 °C and hydrogen addition was continued for an additional 12 hrs. The solution was then filtered to remove the catalyst. The product tested positive for primary amine by a fluorescamine assay.

The above solution was stirred at 25 °C with the slow addition of 0.1 ml thiophosgene (5 hrs). The solution was then evaporated to an orange sticky film. IR (KBr pellet) showed NCS peak at 2106 cm⁻¹. The tertbutyl esters were hydrolyzed and evaporated to dryness.

### Example 2

DICAP (VI) was also prepared by an alternate method. 200 mg (IV) was dissolved in 1 ml water with the addition of 1N NaOH solution to make it basic. 680 mg bromoacetic acid was (pH >8) added gradually and the solution was maintained at 37 °C for 3 days. The solution was negative to a fluorescamine assay. The solution was concentrated and was run down an AG1x8 anion exchange column using a gradient elution of 1M to 7M formic acid. The fractions (3 ml) were lyophilized. The DICAP eluted from fractions 271-304. IR (KBr pellet) 3424 cm⁻¹, 2934 cm⁻¹, 1736 cm⁻¹, 1233 cm⁻¹, 1063 cm⁻¹.

The examples are presented only for the purpose of illustration and not to limit the invention. The invention is intended to encompass conjugation with any peptides, proteins or polymers and any radionuclides or any metals within the full scope of the appended claims.

## Claims

1. A chelating agent having the formula 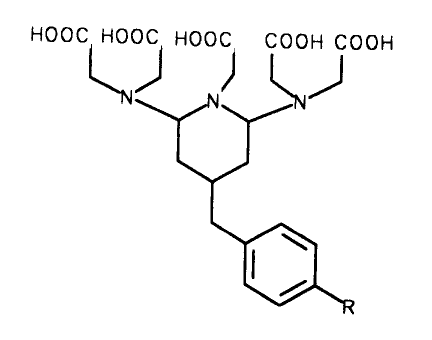 wherein R is selected from the group consisting of -NCS, -NHCOCH₂Br and -N₂Br, or a pharmeucutically acceptable salt thereof.

2. The chelating agent (DICAP) of claim 1, wherein R is -NCS.

3. A complex comprising a radionuclide bound to the chelating agent of claims 1 or 2.

4. A complex comprising a metal bound to the chelating agent of claims 1 or 2.

5. A complex comprising a polypeptide bound to the chelating agent of claims 1 or 2.

6. The complex of claim 5, wherein the polypeptide is an antibody.

7. The complex of claim 5, comprising a radionuclide bound to the chelating agent.
